(19) Europäisches Patentamt
European Patent Office
Office européen des brevets

(11) **EP 4 261 836 A1**

(12) **EUROPEAN PATENT APPLICATION**
published in accordance with Art. 153(4) EPC

(43) Date of publication:
**18.10.2023 Bulletin 2023/42**

(21) Application number: **20964442.6**

(22) Date of filing: **09.12.2020**

(51) International Patent Classification (IPC):
***G16H 20/30*** (2018.01)

(52) Cooperative Patent Classification (CPC):
**G16H 20/30**

(86) International application number:
**PCT/JP2020/045789**

(87) International publication number:
**WO 2022/123666 (16.06.2022 Gazette 2022/24)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Susmed, Inc.
Tokyo 103-0023 (JP)**

(72) Inventor: **UENO Taro
Tokyo 103-0023 (JP)**

(74) Representative: **Vossius & Partner
Patentanwälte Rechtsanwälte mbB
Siebertstraße 3
81675 München (DE)**

(54) **EXERCISE HABITUATION ASSISTING SYSTEM AND EXERCISE HABITUATION ASSISTING PROGRAM**

(57)     A first index information input unit 11 configured to input normal-time subjective index information including a degree of at least one of fatigue, sleep disturbance, and a depressive symptom felt by a patient at normal times when the patient is not exercising, a second index information input unit 12 configured to input objective index information including at least one of normal-time objective index information including at least one of a physical function of the patient at the normal times, a result of diagnosis by a doctor, and treatment content and exercise-time objective index information related to a physical function of the patient during exercise, and an exercise intensity setting unit 13 configured to set exercise intensity based on a plurality of pieces of index information input by the first index information input unit 11 and the second index information input unit 12 are included, and by setting exercise intensity in consideration of a subjective symptom of a patient who frequently complains of a subjective symptom such as fatigue or sleep disturbance even at normal times when the patient is not exercising, it is possible to set appropriate exercise intensity for a patient undergoing treatment for a lifestyle disease by continuing exercise.

**FIG. 2**

EP 4 261 836 A1

**Description**

Technical Field

The present invention relates to an exercise habituation assisting system and an exercise habituation assisting program, and is particularly suitable for use in a system that assists in improvement of a lifestyle through continued exercise.

Background Art

A lifestyle disease is caused by unbalanced diet, lack of exercise, drinking, smoking, stress, etc., continuing for a long period of time. Hyperlipidemia, hypertension, diabetes, etc. are typical lifestyle diseases. Recently, it has been known that many cancers are lifestyle diseases caused by poor lifestyles, and a risk of onset increases with age.

A lifestyle disease is caused by long-term inadequate diet and exercise habits, so in order to prevent and improve the lifestyle disease, it is necessary to record contents of diet and exercise habits over a long period of time and examine content thereof. In addition, it is necessary to report these records to medical personnel such as doctors and receive advice and guidance. Recently, several cases have been reported in which cancer progression has suppressed and cancer tumors have become smaller due to lifestyle improvement.

In response thereto, an apparatus and a system for assisting in lifestyle improvement have been proposed (for example, see PTL 1). PTL 1 discloses a system for selecting and outputting advice for bringing exercise closer to an optimum value for the purpose of cancer prevention/improvement based on historical information of exercise data such as a type and content of exercise, duration of exercise, the amount of exercise (weight $\times$ distance traveled, etc.), quality of exercise, the number of steps, the amount of movement, the amount of body movement, heart rate variability, posture, acceleration, a start and end time of exercise, exercise location, temperature, humidity, presence or absence of an instructor, an apparatus used for exercise, the amount of perspiration before and after exercise, blood pressure, and a change in a blood sugar level.

Examples of advice on exercise provided by the system described in PTL 1 include "After today's meal, you will be sleepy in 30 minutes, so let's take a light walk.", "It's nice weather today, so why don't you walk one station?", "It's raining today, why don't you walk through the underground passages?", "You have some free time today, I'll guide you around the neighborhood.", "Today Kasai Rinkai Park is empty. How about a walk in the aquarium and park?", "Would you like to have your child practice jumping rope?", and "Today you have a weight training practice session at the gym. Let's participate!".

Here, when a patient having a lifestyle disease continues to exercise, one issue is how much exercise load is to be applied. A reason therefor is that, while an effect of improving the lifestyle disease cannot be expected when the exercise load is excessively small, and the patient may become unable to continue exercising or may lose motivation to continue exercising when the exercise load is excessively large.

Note that there have been known systems for assisting in setting of appropriate exercise intensity (for example, see PTL 2 and PTL 3). PTL 2 discloses setting a training menu and exercise intensity in consideration of a training result, heart rate during training, and a subjective degree of fatigue. PTL 3 discloses adjusting exercise load so that the exercise load approaches preset target exercise intensity using objective exercise intensity such as maximal oxygen uptake and heart rate and subjective exercise intensity indicating a degree of toughness of exercise felt by a person being measured.

However, the systems described in PTL 2 and PTL 3 relate to a healthcare field for the purpose of applying appropriate exercise load to a healthy person. In contrast, a patient undergoing treatment for a lifestyle disease frequently complains of a subjective symptom such as fatigue or sleep disturbance even at normal times when the patient is not exercising, and it is inappropriate to adjust exercise intensity using a similar method to that for a healthy person.

PTL 1: JP2019-67447A
PTL 2: JP6725731B
PTL 3: JP2016-32527A

Summary of Invention

Technical Problem

The invention has been made in order to solve such a problem, and an object of the invention is to enable setting of appropriate exercise intensity for a patient undergoing treatment for a lifestyle disease by continuing exercise.

Solution to Problem

To solve the above-mentioned problems, the invention includes a first index information input unit configured to input normal-time subjective index information including a degree of at least one of fatigue, sleep disturbance, and a depressive symptom felt by a patient at normal times when the patient is not exercising, a second index information input unit configured to input objective index information including at least one of normal-time objective index information including at least one of a physical function of the patient at the normal times, a result of diagnosis by a doctor, and treatment content and exercise-time objective index information related to a physical function of the patient during exercise, and an exercise intensity setting unit configured to set exercise intensity based on a plurality of pieces of index information input by the first index information input unit and the second index information input unit.

Advantageous Effects of Invention

According to the invention configured as described above, unlike the conventional technology in the healthcare field in which exercise intensity is set using objective index information during exercise and subjective index information during exercise, exercise intensity is set based on subjective index information of a patient at normal times and objective index information of the patient including at least one of objective index information at normal times and objective index information during exercise. For this reason, it is possible to set exercise intensity in consideration of a subjective symptom of a patient who frequently complains of a subjective symptom such as fatigue or sleep disturbance even at normal times when the patient is not exercising, and it is possible to set appropriate exercise intensity for a patient undergoing treatment for a lifestyle disease by continuing exercise.

Brief Description of Drawings

[FIG. 1] FIG. 1 is a diagram illustrating an overall configuration example of an exercise habituation assisting system according to an embodiment.
[FIG. 2] FIG. 2 is a block diagram illustrating a functional configuration example of a patient terminal according to the present embodiment.
[FIG. 3] FIG. 3 is a diagram illustrating an example of a fatigue input screen according to the present embodiment.
[FIG. 4] FIG. 4 is a schematic diagram illustrating examples of a plurality of exercise programs having different exercise intensities.
[FIG. 5] FIG. 5 is a diagram illustrating an example of an exercise difficulty input screen according to the present embodiment.

Description of Embodiments

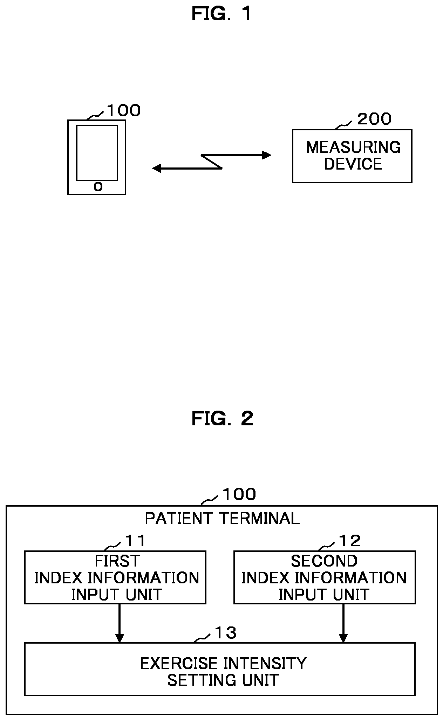

An embodiment of the invention will be described below with reference to the drawings. FIG. 1 is a diagram illustrating an overall configuration example of an exercise habituation assisting system according to the present embodiment. As illustrated in FIG. 1, the exercise habituation assisting system of the present embodiment includes a patient terminal 100 and a measuring device 200, which are wirelessly connected by wireless communication means such as Bluetooth (registered trademark).

The patient terminal 100 includes, for example, a smartphone, a tablet PC, or a personal computer, and an exercise habituation assisting program (hereinafter referred to as an exercise assisting application) is installed therein. The exercise assisting application acquires information measured by the measuring device 200 via wireless communication, and uses the acquired information to perform processing described below, thereby assisting a patient in exercise habituation.

The measuring device 200 has a function of measuring biological information of the patient. For example, the measuring device 200 is a wristwatch-type wearable terminal (so-called smartwatch) and has a function of measuring heart rate of the patient. In the present embodiment, heart rate measured by the measuring device 200 at rest (resting-time heart rate) and heart rate measured by the measuring device 200 during exercise (exercise-time heart rate) are used.

FIG. 2 is a block diagram illustrating a functional configuration example of the patient terminal 100. As illustrated in FIG. 2, the patient terminal 100 includes a first index information input unit 11, a second index information input unit 12, and an exercise intensity setting unit 13 as a functional configuration. These functional blocks 11 to 13 include the exercise assisting application. In practice, the functional blocks 11 to 13 includes a CPU, a RAM, a ROM, etc. of a computer provided in the patient terminal 100, and is implemented by the exercise assisting application stored in a storage medium such as a RAM, a ROM, a hard disk, or a semiconductor memory being operated.

The first index information input unit 11 inputs normal-time subjective index information of the patient at normal times when the patient is not exercising. In the present embodiment, as an example of the subjective index information at normal times, information indicating a degree of fatigue felt by the patient at normal times is input. Fatigue is a tired feeling that makes it difficult to live a normal life. Fatigue is caused by symptoms associated with a disease (pain, anemia, anxiety, and insomnia), side effects of treatment for the disease, etc. The information indicating the degree of fatigue is, for example, information indicating intensity of fatigue.

As an example, the first index information input unit 11 displays a fatigue input screen on a display of the patient terminal 100, and inputs information indicating fatigue through an operation of the patient on the fatigue input screen. For example, a plurality of conditions related to intensity of fatigue is displayed on the fatigue input screen, and the patient selects one of the conditions.

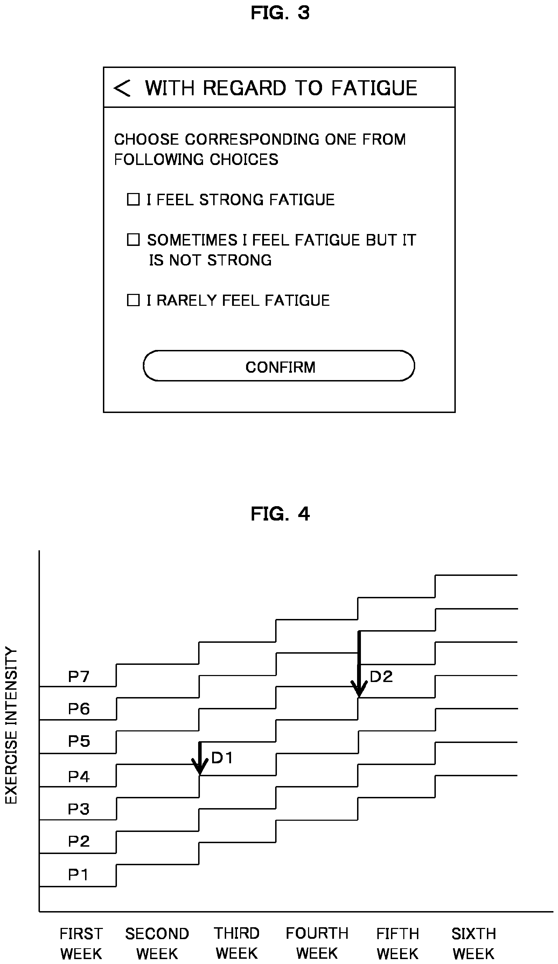

FIG. 3 is a diagram illustrating an example of the fatigue input screen. In the example of FIG. 3, three conditions are illustrated with regard to intensity of fatigue, and a condition on an upper side indicates a condition in which fatigue is stronger. The patient selects and checks one that is closest to a condition of the patient from among the three conditions, and presses a confirmation button, thereby inputting information indicating fatigue to the patient terminal 100.

The second index information input unit 12 inputs objective index information including at least one of normal-time objective index information related to a physical function of the patient at normal times when the patient is not exercising and exercise-time objective index information related to a physical function of the patient during exercise. Here, the normal-time objective index information related to the physical function of the patient at normal times is, for example, resting-time heart rate input from the measuring device 200. The exercise-time objective index information related to the physical function of the patient during exercise is, for example, exercise-time heart rate input from the measuring device 200.

The exercise intensity setting unit 13 sets exercise intensity based on a plurality of pieces of index information input by first index information input unit 11 and the second index information input unit 12. For example, the exercise intensity setting unit 13 prepares a plurality of exercise programs having different exercise intensities as an exercise program to be provided to the patient, sets an exercise program of any of the exercise intensities based on the plurality of pieces of input index information, and presents the exercise program to the patient.

Each of the plurality of exercise programs is a program for performing exercise over a plurality of weeks, and has content in which exercise intensity gradually increases every week. FIG. 4 is a schematic diagram illustrating examples of the plurality of exercise programs having different exercise intensities. FIG. 4 illustrates seven exercise programs P1 to P7. Each of the exercise programs is performed over a period of 6 weeks, and has content in which exercise intensity gradually increases every week.

As an example, the exercise intensity setting unit 13 initially sets exercise intensity based on the objective index information input by the second index information input unit 12 (sets any one of the seven exercise programs illustrated in FIG. 4), and then adjusts the exercise intensity based on subjective index information input by the first index information input unit 11 during treatment for a lifestyle disease.

When initially setting the exercise intensity, the exercise intensity setting unit 13 uses, for example, normal-time objective index information (resting-time heart rate) and exercise-time objective index information (exercise-time heart rate) to calculate exercise intensity (%HHR) based on predicted maximum heart rate as follows, and sets any one of the exercise programs based on a value of %HHR.

$$\%HHR = (\text{Exercise-time maximum heart rate} - \text{resting-time heart rate})/(HHR - \text{resting-time heart rate}) \times 100$$

where, HHR (estimated maximum heart rate) = 220 - age

Note that, even though an example of initially setting exercise intensity by calculating %HHR has been described here, the invention is not limited thereto. For example, the exercise intensity may be initially set based only on the normal-time objective index information (resting-time heart rate), or the exercise intensity may be initially set based only on the exercise-time objective index information (exercise-time heart rate).

After initially setting the exercise intensity as described above, the exercise intensity setting unit 13 adjusts the exercise intensity as necessary based on the subjective index information input by the first index information input unit 11. As an example, the exercise intensity setting unit 13 adjusts the exercise intensity based on normal-time subjective index information (information indicating a degree of fatigue). The exercise intensity is adjusted, for example, by changing the exercise program.

For example, when a condition of the strongest fatigue among the three conditions illustrated with regard to intensity of fatigue as in FIG. 3 is input, the exercise intensity setting unit 13 lowers currently set exercise intensity by two levels. In addition, when a condition of the second strongest fatigue is input, the exercise intensity setting unit 13 lowers the currently set exercise intensity by one level. On the other hand, when a condition of the weakest fatigue is input, the exercise intensity setting unit 13 maintains the currently set exercise intensity.

Exercise intensity is adjusted, for example, every week. FIG. 4 illustrates an example D1 in which the exercise intensity is lowered by one level at the beginning of the third week (an example in which an exercise program P4 is changed to an exercise program P3 having exercise intensity one level lower than that of the exercise program P4), and an example D2 in which the exercise intensity is lowered by two levels at the beginning of the fifth week (an example in which an exercise program P6 is changed to the exercise program P4 having exercise intensity two levels lower than that of the exercise program P6).

As described above in detail, according to the present embodiment, unlike the conventional technology in the healthcare field in which exercise intensity is set using objective index information during exercise and subjective index information during exercise, exercise intensity is set based on subjective index information of a patient at normal times (information indicating a degree of fatigue) and objective index information of the patient including at least one of objective index information at normal times and objective index information during exercise. For this reason, it is possible to set exercise intensity in consideration of a subjective symptom of a patient who frequently complains of fatigue even at normal times when the patient is not exercising, and it is possible to set appropriate exercise intensity for a patient undergoing treatment for a lifestyle disease by continuing exercise.

Note that, in the above embodiment, a description has been given of an example in which index information related to a physical function of a patient, specifically, resting-time heart rate input from the measuring device 200 is used as objective index information of the patient at normal times when the patient is not exercising. However, the invention is not limited thereto. For example, information related to leg muscle strength, 6-minute walking distance, walking speed, chair rise test, grip strength, timed-up & go test, 2-step test, functional reach test, body composition, cognitive function test, and nutritional status (diet, etc.) may be used as index information related to the physical function of the patient.

In addition, as the normal-time objective index information of the patient, the second index information input unit 12 may input index information related to either a diagnosis result by a doctor or content of treatment instead of the above-described index information related to the physical function of the patient. Alternatively, any two or three pieces of information of the physical function of the patient, the diagnosis result by the doctor, and the content of treatment may be input. When a plurality of pieces of information is input, for example, the input information may be used to calculate a score by a predetermined function, and the score may be used as normal-time objective index information.

Here, the normal-time objective index information related to the diagnosis result by the doctor is, for example, information related to seriousness of a symptom or severity of a nutritional status (diet, etc.). For example, in the case of a cancer patient, the normal-time objective index information is information indicating stages (five levels from 0 to 4) classified according to progress of cancer. Alternatively, the normal-time objective index information may be information indicating a result of diagnosis made by a doctor with regard to a renal function or a liver function. The normal-time objective index information with regard to treatment content is, for example, information indicating content of chemotherapy (drug therapy). A physical or mental burden on the patient changes depending on what type of drug is administered at what amount or frequency. The exercise intensity setting unit 13 may initially set the exercise intensity based on above normal-time objective index information.

Further, in the above embodiment, a description has been given of an example of inputting information indicating a degree of fatigue as subjective index information of the patient at normal times when the patient is not exercising. However, the invention is not limited thereto. For example, the first index information input unit 11 may input information related to a degree of sleep disturbance or a depressive symptom felt by the patient at normal times as the normal-time subjective index information of the patient. Alternatively, information related to degrees of two or three of fatigue, sleep disturbance, and the depressive symptom may be input. When a plurality of pieces of information is input, for example, a score may be calculated by a predetermined function using the input information, and the score may be used as the normal-time subjective index information. The exercise intensity setting unit 13 may adjust above exercise intensity based on the normal-time subjective index information.

Further, in the above embodiment, a description has been given of an example in which the exercise intensity is adjusted only in a direction of lowering the exercise intensity based on the normal-time subjective index information (information indicating a degree of fatigue). However, the invention is not limited thereto. For example, the exercise intensity may be adjusted both in a direction of raising the exercise intensity and the direction of lowering the exercise intensity based on the normal-time subjective index information. For example, according to information indicating fatigue input by the patient every week, the exercise intensity may be adjusted in a direction of strengthening the exercise intensity when the information indicates that fatigue has improved compared to a previous week, while the exercise intensity may be adjusted in a direction of weakening the exercise intensity when the information indicates that fatigue has worsened compared to the previous week. At this time, a degree of increase or decrease in exercise intensity may be adjusted according to a degree of change in fatigue (the intensity is changed by one level when the degree of change in fatigue is less than a threshold, and the exercise intensity is changed by two levels when the degree of change in fatigue is larger than the threshold).

Further, in the above embodiment, a description has been given of an example in which the exercise intensity after initial setting is adjusted based on the normal-time subjective index information (information indicating a degree of fatigue) of the patient at normal times when the patient is not exercising. However, the invention is not limited thereto. For example, the exercise intensity after initial setting may be adjusted based on the normal-time subjective index information and exercise-time subjective index information related to difficulty of exercise felt by the patient during exercise.

In this case, the first index information input unit 11 inputs the normal-time subjective index information and the exercise-time subjective index information. The exercise-time subjective index information related to the difficulty of exercise felt by the patient during exercise is, for example, information input by displaying an exercise difficulty input screen on the display of the patient terminal 100 and operating the exercise difficulty input screen by the patient. For example, the first index information input unit 11 displays a plurality of conditions related to the difficulty of exercise on the exercise difficulty input screen, and allows the patient to select a corresponding condition from the conditions.

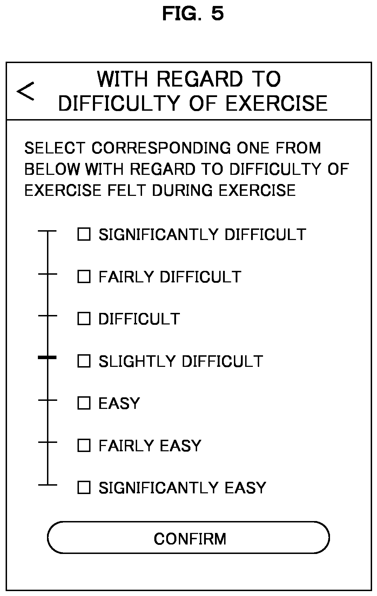

FIG. 5 is a diagram illustrating an example of the exercise difficulty input screen. In the example of FIG. 5, seven conditions are illustrated with regard to difficulty of exercise, and an upper one indicates a condition in which exercise is more difficult. The patient selects and checks one most closely matching a condition of the patient from the seven conditions during exercise, and press a confirmation button, thereby inputting information indicating difficulty of exercise to the patient terminal 100.

For example, the exercise intensity setting unit 13 adjusts exercise intensity for a subsequent week and thereafter based on the normal-time subjective index information input through the fatigue input screen illustrated in FIG. 3 and the exercise-time subjective index information input through the exercise difficulty input screen illustrated in FIG. 5. As a conceivable example of an adjustment method, a score may be calculated by a predetermined function using the normal-time subjective index information and the exercise-time subjective index information, and the exercise intensity after initial setting may be adjusted based on the score.

As another example, an increase/decrease range of exercise intensity may be tentatively determined based on the normal-time subjective index information, an increase/decrease range of exercise intensity may be tentatively determined based on the exercise-time subjective index information, and a final increase/decrease range may be determined by taking into the two tentatively determined increase/decrease ranges. For example, when both the increase/decrease ranges match in a direction of raising the exercise intensity or in a direction of lowering the exercise intensity, a larger one of the increase/decrease ranges is adopted. On the other hand, when increase/decrease directions do not match, both the increase/decrease ranges are canceled out (for example, when the increase/decrease range of the exercise intensity based on the normal-time subjective index information is two levels down, and the increase/decrease range of the exercise intensity based on the exercise-time subjective index information is one level up, the increase/decrease range of the exercise intensity is set to one level down).

Note that, when the increase/decrease range of the exercise intensity is tentatively set based on information related to seven levels of difficulty of exercise input through the exercise difficulty input screen illustrated in FIG. 5, for example, the following procedure may be adopted. In the exercise difficulty input screen of FIG. 5, when a most difficult condition is input, the currently set exercise intensity is lowered by two levels. In addition, when second and third most difficult conditions are input, the currently set exercise intensity is lowered by one level. In addition, when an easiest condition is input, the currently set exercise intensity is raised by two levels. In addition, when second and third easiest conditions are input, the currently set exercise intensity is raised by one level. However, this is only an example. For example, a degree of increase/decrease in exercise intensity may be adjusted according to a degree of change in difficulty of exercise input every week.

Further, in the above embodiment, the objective index information (normal-time objective index information and exercise-time objective index information) input by the second index information input unit 12 is used only when the exercise intensity is initially set. However, the invention is not limited thereto. For example, the exercise intensity setting unit 13 may initially set the exercise intensity based on the objective index information input by the second index information input unit 12 (for example, a result of diagnosis by a doctor, treatment content, etc.), determine necessity of adjusting the exercise intensity based on the objective index information input by the second index information input unit 12 (for example, %HHR, etc.) during treatment for a lifestyle disease thereafter, and adjust the exercise intensity based on the subjective index information input by the first index information input unit 11 when it is determined that the exercise intensity needs to be adjusted.

According to this method, even during treatment for the lifestyle disease after initial setting of the exercise intensity, the exercise intensity is adjusted using both the subjective index information and the objective index information of the patient, and thus the exercise intensity can be adjusted more appropriately. In this method, when %HHR suggests that a symptom is improving or worsening through treatment for a lifestyle disease, or when %HHR suggests that a symptom is not improving, the exercise intensity can be appropriately adjusted based on normal-time subjective index information indicating fatigue, etc. of the patient or exercise-time subjective index information indicating difficulty, etc.

during exercise.

Alternatively, the exercise intensity setting unit 13 may initially set the exercise intensity based on the objective index information input by the second index information input unit 12 (for example, a result of diagnosis by a doctor, treatment content, etc.), determine necessity of adjusting the exercise intensity based on the subjective index information input by the first index information input unit 11 during treatment for a lifestyle disease thereafter, and adjust the exercise intensity based on the objective index information input by the second index information input unit 12 (for example, %HHR, etc.) when it is determined that the exercise intensity needs to be adjusted.

Even in this method, during treatment for the lifestyle disease after initial setting of the exercise intensity, the exercise intensity is adjusted using both the subjective index information and the objective index information of the patient, and thus the exercise intensity can be adjusted more appropriately. According to this method, during treatment for the lifestyle disease, when fatigue, sleep disturbance, a depressive symptom, etc. of the patient is improving or worsening, or when %HHR suggests that a symptom is not improving, the exercise intensity can be appropriately adjusted based on objective index information such as %HHR.

Further, in the above embodiment, a description has been given of an example in which the exercise intensity is adjusted based on the subjective index information after initially setting the exercise intensity based on the objective index information. However, the reverse may be adopted. That is, the exercise intensity setting unit 13 may initially set the exercise intensity based on the normal-time subjective index information input by the first index information input unit 11, and adjust the exercise intensity based on the objective index information input by the second index information input unit 12 during treatment for the lifestyle disease thereafter.

Further, in the above embodiment, a description has been given of an example in which the patient terminal 100 and the measuring device 200 are wirelessly connected to each other, and information measured by the measuring device 200 is wirelessly transmitted to the patient terminal 100. However, the invention is not limited thereto. For example, the patient may view information displayed on the measuring device 200, and operate an input device such as a keyboard or a touch panel of the patient terminal 100 to input measurement information.

Further, in the above embodiment, a description has been given of an example in which the patient terminal 100 has functions of the first index information input unit 11, the second index information input unit 12, and the exercise intensity setting unit 13. However, the invention is not limited thereto. For example, a server apparatus connected to a communication network such as the Internet or a mobile phone network may have these functions, and information on the exercise intensity set in the server apparatus may be transmitted to the patient terminal 100 and set in the patient terminal 100. Alternatively, the information on the exercise intensity set in the server apparatus may be configured to be allowed to be read from the patient terminal 100.

In addition, the above embodiment merely illustrates an example of implementation in carrying out the invention, and the technical scope of the invention should not be construed in a limited manner by the embodiment. Thus, the invention may be carried out in various forms without departing from spirit or essential characteristics thereof.

Reference Signs List

11: first index information input unit
12: second index information input unit
13: exercise intensity setting unit
100: patient terminal
200: measuring device

**Claims**

1. An exercise habituation assisting system **characterized by** comprising:

a first index information input unit configured to input normal-time subjective index information including a degree of at least one of fatigue, sleep disturbance, and a depressive symptom felt by a patient at normal times when the patient is not exercising;
a second index information input unit configured to input objective index information including at least one of normal-time objective index information including at least one of a physical function of the patient at the normal times, a result of diagnosis by a doctor, and treatment content and exercise-time objective index information related to a physical function of the patient during exercise; and
an exercise intensity setting unit configured to set exercise intensity based on a plurality of pieces of index information input by the first index information input unit and the second index information input unit.

2. The exercise habituation assisting system according to claim 1, **characterized in that** the exercise intensity setting unit initially sets the exercise intensity based on the objective index information input by the second index information input unit, and adjusts the exercise intensity based on the normal-time subjective index information input by the first index information input unit during treatment for a lifestyle disease thereafter.

3. The exercise habituation assisting system according to claim 2, **characterized in that**:

   the first index information input unit inputs the normal-time subjective index information and exercise-time subjective index information related to difficulty of exercise felt by the patient during exercise; and
   the exercise intensity setting unit adjusts the exercise intensity based on the normal-time subjective index information and the exercise-time subjective index information input by the first index information input unit during the treatment for the lifestyle disease after initial setting of the exercise intensity.

4. The exercise habituation assisting system according to any one of claims 1 to 3, **characterized in that** the exercise intensity setting unit initially sets the exercise intensity based on the objective index information input by the second index information input unit, determines necessity of adjusting the exercise intensity based on the objective index information input by the second index information input unit during the treatment for the lifestyle disease thereafter, and adjusts the exercise intensity based on subjective index information input by the first index information input unit when it is determined that the exercise intensity needs to be adjusted.

5. The exercise habituation assisting system according to claim 1, **characterized in that** the exercise intensity setting unit initially sets the exercise intensity based on the objective index information input by the second index information input unit, determines necessity of adjusting the exercise intensity based on the normal-time subjective index information input by the first index information input unit during the treatment for the lifestyle disease thereafter, and adjusts the exercise intensity based on the objective index information input by the second index information input unit when it is determined that the exercise intensity needs to be adjusted.

6. The exercise habituation assisting system according to claim 5, **characterized in that**:

   the first index information input unit inputs the normal-time subjective index information and exercise-time subjective index information related to difficulty of exercise felt by the patient during exercise; and
   the exercise intensity setting unit determines necessity of adjusting the exercise intensity based on the normal-time subjective index information and the exercise-time subjective index information input by the first index information input unit during the treatment for the lifestyle disease after initial setting of the exercise intensity.

7. The exercise habituation assisting system according to claim 1, **characterized in that** the exercise intensity setting unit initially sets the exercise intensity based on the normal-time subjective index information input by the first index information input unit, and adjusts the exercise intensity based on the objective index information input by the second index information input unit during the treatment for the lifestyle disease thereafter.

8. An exercise habituation assisting program for causing a computer to function as:

   a first index information input means configured to input normal-time subjective index information including a degree of at least one of fatigue, sleep disturbance, and a depressive symptom felt by a patient at normal times when the patient is not exercising;
   a second index information input means configured to input objective index information including at least one of normal-time objective index information including at least one of a physical function of the patient at the normal times, a result of diagnosis by a doctor, and treatment content and exercise-time objective index information related to a physical function of the patient during exercise; and
   an exercise intensity setting means configured to set exercise intensity based on a plurality of pieces of index information input by the first index information input means and the second index information input means.

FIG. 1

FIG. 2

## FIG. 3

```
< WITH REGARD TO FATIGUE

CHOOSE CORRESPONDING ONE FROM
FOLLOWING CHOICES

  ☐ I FEEL STRONG FATIGUE

  ☐ SOMETIMES I FEEL FATIGUE BUT IT
    IS NOT STRONG

  ☐ I RARELY FEEL FATIGUE


         ( CONFIRM )
```

## FIG. 4

FIG. 5

WITH REGARD TO
DIFFICULTY OF EXERCISE

SELECT CORRESPONDING ONE FROM
BELOW WITH REGARD TO DIFFICULTY OF
EXERCISE FELT DURING EXERCISE

☐ SIGNIFICANTLY DIFFICULT

☐ FAIRLY DIFFICULT

☐ DIFFICULT

☐ SLIGHTLY DIFFICULT

☐ EASY

☐ FAIRLY EASY

☐ SIGNIFICANTLY EASY

CONFIRM

**INTERNATIONAL SEARCH REPORT**

| International application No. |
|---|
| PCT/JP2020/045789 |

A. CLASSIFICATION OF SUBJECT MATTER
Int. Cl. G16H20/30(2018.01)i
FI: G16H20/30

According to International Patent Classification (IPC) or to both national classification and IPC

B. FIELDS SEARCHED

Minimum documentation searched (classification system followed by classification symbols)
Int. Cl. G16H10/00-80/00, G06Q10/00-99/00

Documentation searched other than minimum documentation to the extent that such documents are included in the fields searched
Published examined utility model applications of Japan    1922-1996
Published unexamined utility model applications of Japan  1971-2021
Registered utility model specifications of Japan          1996-2021
Published registered utility model applications of Japan  1994-2021

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2013-163010 A (INDUSTRIAL TECHNOLOGY RESEARCH INSTITUTE) 22 August 2013 (2013-08-22), paragraphs [0009], [0014], [0016], [0017], [0036], fig. 1, 2 | 1-8 |
| Y | JP 2018-166885 A (SHOJI, Yusuke) 01 November 2018 (2018-11-01), paragraph [0060], fig. 6 | 1-8 |
| Y | JP 2004-54591 A (INSTITUTE OF TSUKUBA LIAISON CO., LTD.) 19 February 2004 (2004-02-19), paragraph [0028], fig. 3 | 1-8 |

☒ Further documents are listed in the continuation of Box C.　　☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 02.03.2021 | 16.03.2021 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

<table>
<tr><td colspan="3" align="center">**INTERNATIONAL SEARCH REPORT**</td><td>International application No.<br>PCT/JP2020/045789</td></tr>
</table>

C (Continuation).    DOCUMENTS CONSIDERED TO BE RELEVANT

| Category* | Citation of document, with indication, where appropriate, of the relevant passages | Relevant to claim No. |
|---|---|---|
| Y | 本山貢．運動療法．日本臨牀．vol. 57, no. 7, Hiro Hayafuji, K.K. Nippon-Rinsho-sha, 31 July 1991, pp. 135(1595)-141(1601), p. 138, left column, line 25 to right column, line 15, non-official translation (MOTOYAMA, Mitsugi, SASAKI, Jun. Exercise therapy. Japanese Journal of Clinical Medicine.) | 3-7 |
| Y | JP 2016-32527 A (PANASONIC INTELLECTUAL PROPERTY MANAGEMENT CO., LTD.) 10 March 2016 (2016-03-10), paragraphs [0072]-[0088], fig. 9-13 | 3-7 |
| Y | WO 2019/146616 A1 (NIPPON TELEGRAPH AND TELEPHONE CORP.) 01 August 2019 (2019-08-01), paragraphs [0013]-[0018], fig. 1 | 4-7 |
| Y | WO 2018/021435 A1 (MITOS CO., LTD.) 01 February 2018 (2018-02-01), paragraph [0018], fig. 1 | 4-7 |
| A | JP 2016-134131 A (SEIKO EPSON CORP.) 25 July 2016 (2016-07-25), entire text, all drawings | 1-8 |
| A | JP 2017-148544 A (FUKUDA DENSHI CO., LTD.) 31 August 2017 (2017-08-31), entire text, all drawings | 1-8 |

Form PCT/ISA/210 (continuation of second sheet) (January 2015)

INTERNATIONAL SEARCH REPORT
Information on patent family members

International application No.

PCT/JP2020/045789

| Patent Documents referred to in the Report | Publication Date | Patent Family | Publication Date |
|---|---|---|---|
| JP 2013-163010 A | 22.08.2013 | US 2013/0209978 A1 paragraphs [0017], [0022], [0024], [0025], [0043], fig. 1, 2 TW 201332534 A | |
| JP 2018-166885 A | 01.11.2018 | (Family: none) | |
| JP 2004-54591 A | 19.02.2004 | (Family: none) | |
| JP 2016-32527 A | 10.03.2016 | (Family: none) | |
| WO 2019/146616 A1 | 01.08.2019 | (Family: none) | |
| WO 2018/021435 A1 | 01.02.2018 | (Family: none) | |
| JP 2016-134131 A | 25.07.2016 | (Family: none) | |
| JP 2017-148544 A | 31.08.2017 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019067447 A
- JP 6725731 B
- JP 2016032527 A